# EUROPÄISCHE PATENTSCHRIFT

(11) **EP 0 570 331 B1**
(45) Veröffentlichungstag und Bekanntmachung des Hinweises auf die Patenterteilung: **10.09.1997**
(21) Anmeldenummer: 93810297.7
(22) Anmeldetag: 22.04.1993
(51) Int. Cl.: A61F 2/06, A61L 27/00

(54) **Verfahren und Einrichtung zur Herstellung von vaskulären Endoprothesen**
Method and apparatus to make vascular prostheses
Méthode et appareil pour obtenir des prothèses vasculaires

(30) Priorität: 11.05.1992 CH 1498/92
(43) Veröffentlichungstag der Anmeldung: 18.11.1993
(73) Patentinhaber: SULZER Medizinaltechnik AG, 8404 Winterthur (CH)
(72) Erfinder: Mueller-Glauser, Werner, CH-8542 Wiesendangen (CH); Rieser, Franz, CH-8542 Wiesendangen (CH); Bittmann, Pedro, CH-8057 Zürich (CH); Dardel, Eric, CH-8472 Seuzach (CH)
(74) Vertreter: Frei, Alexandra Sarah

(56) Entgegenhaltungen:
- EP-A- 248 247
- EP-A- 393 788
- EP-A- 399 340
- EP-A- 444 270
- EP-A- 462 051
- US-A- 5 035 708
- US-A- 5 171 261

## Beschreibung

Die Erfindung betrifft ein Verfahren und eine Einrichtung gemäss den Oberbegriffen der entsprechenden unabhängigen Ansprüche zur Herstellung von Endoprothesen für eine wenigstens teilweise direkte Berührung mit dem Blut oder mit der Lymphe, wie beispielsweise Blut- oder Lymphgefässprothesen, Herzklappen, Kunstherzen und dergleichen, wobei eine körpereigene Zellen enthaltende Suspension hergestellt und ein vorgeformtes, künstliches Trägermaterial mit dieser Suspension behandelt wird. Ein solches Verfahren ist z.B. aus der EP-A-0 393 788 bekannt. Durch die Implantation derartiger Endoprothesen kann die Neubildung von Teilen des kardiovaskulären Systems bei Mensch und Tier unterstützt werden.

Endoprothesen, die mehrheitlich oder auch nur teilweise in direktem Kontakt mit Blut oder Lymphe stehen, müssen ganz spezielle Anforderungen erfüllen, wie sie bei Endoprothesen, die nur mit dem Gewebe verwachsen müssen, nicht gestellt sind. Native Blut- oder Lymphgefässe oder andere blut- oder lympheführende Hohlräume sind an ihren Innenflächen mit einer speziellen Zellschicht bedeckt, dem Endothelium. Zwischen Blut und Endothelzellen herrscht eine völlige Abstimmung, die dem Blut (oder der Lymphe) seine volle, bekanntermassen auch sehr komplexe normale Funktionsfähigkeit erhält, bzw. eine Gerinnung verhindert. Bis anhin erfüllten Endoprothesen, die in Blutbahnen eingesetzt wurden, diese Aufgabe nur mässig. Man kann davon ausgehen, dass synthetische Materialien trotz intensivster Forschung in dieser Hinsicht im Langzeiteinsatz nur als suboptimal angesehen werden können und das Blut (oder die Lymphe) einem zusätzlichen Stress aussetzen, der bei nativem Gewebe, dem Endothelium, nicht auftritt.

Spätestens seit dem Jahr 1978, in dem die Arbeiten von Herring et al. (A single-staged technique for seeding vascular grafts with autogenous endothelium, Surgery 84:498-504, 1978) veröffentlicht wurden, ist bekannt, dass körpereigene Endothelzellen, die z.B. aus Venen gewonnen werden, einen gerinnungshemmenden Belag bilden, wenn sie auf die Innenoberfläche von Gefässprothesen aufgetragen und nach der Implantation erneut dem Blustrom ausgesetzt werden.

Da bei der Gewinnung von Endothelzellen aus Venen in der Regel nur eine Entnahme von Abschnitten mit sehr begrenzter Länge (typischerweise 5 bis 10 cm) möglich ist, muss zwischen der Entnahme des Venen-Stücks und der Implantation der beschichteten Prothese ein Schritt zur Vermehrung der entnommenen körpereigenen Endothelzellen eingeschoben werden, der im Zellkulturlabor durchgeführt wird und in der Regel ein bis drei Wochen dauert. Dieser Zwischenschritt birgt beträchtliche Gefahren in sich. Zum einen muss während der ganzen Zeit der in-vitro-Kultivierung eine absolute Sterilität gewahrt bleiben, um weder die kultivierten Zellen noch anschliessend den Patienten durch eine Infektion zu gefährden. Zum anderen verändern sich die Zellen bei einer derartigen Vermehrung, wobei eine sogenannte Dedifferenzierung stattfindet, die mit den Bedingungen der in-vitro-Kultur einhergehen und in deren Verlauf die Zellen im allgemeinen in einen weniger spezialisierten Zustand zurückfallen, für den es keine genaue Entsprechung im Körper gibt. Daher ist die Frage berechtigt, in wie weit die nach einer Kulturperiode mit vielen Vermehrungsschritten unter künstlichen Bedingungen entstandenen Zellen, die zwar von Zellen aus dem Körper des Patienten abstammen, bei der Prothesen-Implantation bezüglich ihrer Funktionen noch wirklich körpereigenen Zeilen entsprechen.

Es ist das Verdienst von Jarrell et al. (Use of freshly isolated capillary endothelial cells for the immediate establishment of a monolayer on a vascular graft at surgery, Surgery 100:392-399, 1986), Schmidt et al. (Micrcrovascular endothelial cell seeding of small diameter Dacron® vascular grafts, J.Invest Surg., 1:35-44, 1988), Pearce et al. (Successfull endothelial seeding with omentally derived microvascular endothelial cells, J.Vasc. Surg., 5:203-210, 1987) und von anderen Autoren, mittels Tierexperimenten gezeigt zu haben, dass Endothelzellen, die aus Mikrogefässen isoliert werden, sich anpassen und die Funktion von arteriellen Endothelzellen übernehmen können, wenn sie in Arterien-Prothesen ausgesät werden, in denen bspw. die physiologische Strömungsgeschwindigkeit des Blutes deutlich höher ist als im mikrovaskulären Teil des Gefässnetzes, aus dem die Zellen isoliert worden sind. Es ist also bekannt, dass sich in Gefässprothesen, die mit mikrovaskulären Endothelzellen besät sind, beispielsweise bei Hunden eine funktionelle, gerinnungshemmende Auskleidung bildet. Die mikrovaskulären Endothelzellen können beispielsweise aus Fettgewebe gewonnen werden, bspw. durch Entnahme eines Teils des Omentums oder einer begrenzten Menge von subkutanem Fettgewebe.

Ähnliche Verfahren und Vorrichtungen zu deren Durchführung sind auch beschrieben in den Publikationen EP-A-0 399 340 und US-A-5 035 708.

Es hat sich in der praktischen Durchführung gezeigt, dass je Gramm des entnommenen Fettgewebes gewöhnlich etwa 100'000, gelegentlich auch 1'000'000 vitale mikrovaskuläre Zellen gewonnen werden können. Geht man davon aus, dass eine Prothese mit einem Innendurchmesser von 6 mm und einer Länge von 100 cm eine Oberfläche von etwa 200 cm² aufweist, und dass eine Versorgung der Oberfläche mit ungefähr 100'000 Zellen pro cm² genügt, um die athrombogene Schicht nach Implantation neu zu generieren, so zeigt eine einfache Rechnung, dass mit der Entnahme von maximal 200 g Gewebe genügend Zellmaterial gewonnen werden kann, um die Beschichtung eines vollständigen Bypasses von der Leistengegend bis zum Fuss vornehmen zu können. Das bedeutet dann aber, dass die oben beschriebene in-vitro-Vermehrung zur Produktion der für die Prothesenbeschichtung notwendigen Anzahl von Endothelzellen wegfallen kann, d.h. mikrovaskuläre körpereigene Endothelzellen können in solchen Mengen gewonnen werden, dass die Prothese anschliessend an die Gewebeentnahme sofort fertiggestellt werden könnte, wenn nur ein genügend schnelles Verfahren für die Isolation der Zellen zur Verfügung stehen würde.

Während für die Methode mit in-vitro-Vermehrung nach Mansfield et al. (Preventing thrombus on artificial vascular surfaces: True endothelial cell linings, Trans.Am.Soc.Artif.Intern.Organs., 21:264-272, 1975) und allen späteren Autoren zwischen der Entnahme von Teilen grosser Gefässe (Arterien, Venen) zur Gewinnung von vaskulären körpereigenen Endothelzellen aus dem Patienten und der Prothesen-Implantation ein bis drei oder mehr Wochen vergehen müssen (je nach Effizienz des Kultursystems), gelingt es bei der Verwendung von mikrovaskulären Endothelzellen gemäss Schmidt et al. (J.Invest.Surg., 1:35-44, 1988), diese Zeitspanne auf drei Stunden zu reduzieren, weil der Schritt der Zellvermehrung wegfällt. Dennoch hat auch dieses Verfahren nicht Eingang gefunden in die chirurgische Routine, insbesondere weil es trotzdem nicht schnell genug ist. Bei einer Koronar-Bypass-Operation werden noch heute mit Vorteil patienteneigene Gefässe zur Überbrückung der Gefäss-Verengungen verwendet. Dabei rechnet der Chirurg mit einer Zeitspanne von ungefähr einer Stunde zwischen dem Augenblick, in dem bspw. die Entahme von Venen am Bein begonnen wird, und dem Moment, in dem die vorbereitete Vene zum Einnähen in die Herzgegend bereit steht.

Das bekannte Verfahren zur Herstellung von Endoprothesen ist im Detail das folgende.

In einem ersten Verfahrensschritt werden aus dem bevorzugt subkutanen Fettgewebe eines Patienten, das neben den mikrovaskulären Zellen eine Menge anderer Zellen und nichtzellulären Gewebekomponenten enthält, mikrovaskuläre Zellen isoliert und diese in einem weiteren Verfahrensschritt auf bestimmte Flächenteile eines vorgeformten künstlichen Trägers aufgetragen. Es hat sich gezeigt, dass sich nach der Implantierung derartig hergestellter Prothesen im Verlaufe von Wochen bis Monaten auf den mit dem Blut permanent in Kontakt stehenden Flächenteilen ein Gewebe entwickelt, das dem Endothelium nativer Gefässe ähnlich ist und auf einer Gewebeschicht liegt, die ebenfalls der nativen Arterienwand ähnlich ist (Schmidt et al., J.Invest. Surg., 1:35-44, 1988). Diese Gewebe, die sich beispielsweise auf der Innenseite von Gefässprothesen bilden, werden daher auch als Neoarterie bezeichnet. Die Neoarterie ist wie die nativen Arterie vollständig mit athrombogen Endothelzellen bedeckt, sodass das Blut in der Prothese nicht gerinnt bzw. seine volle und ungestörte Funktionsfähigkeit entfalten kann.

Der erste Verfahrensschritt besteht aus der Isolierung der für die Einlagerung erwünschten mikrovaskulären Zellen durch mechanische Zerkleinerung des aus dem Patienten entnommenen Gewebes, durch darauffolgenden enzymatischen Aufschluss Verdauung des zerkleinerten Gewebes mit einem dazu geeigneten Enzym und aus der Abtrennung möglichst aller nicht erwünschten Bestandteile aus der durch den Aufschluss hergestellten Suspension zur Isolation und Aufkonzentrierung der mikrovaskulären Zellen.

In den Arbeiten von Schmidt et al. (J.Invest.Surg., 1:35-44, 1988) und Jarrell et al. (Surgery, 100:392-399, 1986) wird für diese Isolation anschliessend an die enzymatische Verdauung ein Schritt der Zell-Reinigung und/oder der Zell-Sortierung eingefügt. Die durch die Verdauung gewonnene Zellsuspension wird in einem oder mehreren Zentrifugations-Schritten - unter Umständen sogar unter Zuhilfenahme eines sogenannten Dichtegradienten - in die verschiedenen Zelltypen separiert und von nicht zellulären Gewebekomponenten befreit. Durch Zentrifugation können beispielsweise die spezifisch leichteren, fetthaltigen Adipozyten von den Endothelzellen, die ein grösseres spezifisches Gewicht haben, getrennt werden.

Für die Beschichtung der Prothese mit den isolierten Zellen werden diese in an sich bekannter Weise entweder durch Filtration der Suspension durch eine poröse Prothese oder durch Zentrifugation in einer schnell rotierenden Prothese (Publikation EP-462051) in der entsprechend ausgestalteten Oberfläche der Prothese eingelagert.

Ein schnelleres Verfahren wird in der Publikation EP-A-0 393 788 beschrieben. Es werden dabei durch Zerschneiden mit einem chirurgischen Messer hergestellte Gewebepartikel auf poröse Prothesen aufgebracht. Das Verfahren ist kaum geeignet für den humanen Fall.

Es ist nun die Aufgabe der Erfindung, das oben beschrieben Verfahren zur Herstellung einer Endoprothese durch Herstellung einer körpereigene Zellen enthaltenden Suspension und darauffolgender Behandlung eines vorgeformten, künstlichen Trägermaterials mit dieser Suspension derart zu verbessern, dass sie für die Durchführung im Operationssaal und für die Humanmedizin geeignet werden. Das gesamte Verfahren der Herstellung der Suspension und der darauffolgenden Behandlung der Prothese mit der Suspension soll dabei nicht mehr Zeit beanspruchen als die chirurgischen Vorbereitungen für die Implantation der Prothese. Das heisst mit anderen Worten, es soll unter Verwendung des erfindungsgemässen Verfahrens möglich sein, beispielsweise eine Endoprothese mit eingelagerten körpereigenen, mikrovaskulären Endothelzellen zu implantieren, wobei die verwendeten körpereigenen Zellen während derselben Operation beispielsweise in Form von subkutanem Fettgewebe entnommen werden können. Das bekannte Verfahren soll also derart verbessert werden, dass eine Stunde für die Herstellung der Suspension und die darauffolgende Behandlung der Prothese mit der Suspension genügt und dass die nach dem erfindungsgemässen Verfahren hergestellte Prothese die Regeneration des Endothels in mindestens gleichem Masse unterstützt wie Prothesen, die nach Verfahren gemäss dem Stande der Technik hergestellt werden. Es handelt sich dabei um das technische Problem, das Transplantationsmaterial in einer minimalen Anzahl von möglichst einfachen Verfahrensschritten mit möglichst wenigen Manipulationen aufzubereiten aber trotzdem mit der sicheren Gewähr, dass die transplantierten Zellen ihre Aufgabe nach der Transplantation optimal zu erfüllen fähig sind.

Die gestellte Aufgabe zur Verbesserung des oben beschriebenen, bekannten Verfahrens wird gelöst durch das Verfahren zur Herstellung von insbesondere in der Humanmedizin anwendbaren Endoprothesen, welche Berührungsflächen für den direkten Kontakt mit Blut und/oder Lymphe und an diesen Berührungsflächen angelagerte, eine Regeneration einer lebenden Oberflächenschicht initiierende Zellen und Zellverbände aufweisen, wobei das Verfahren daraus besteht, aus körpereigenem Gewebe, das neben den für die Regeneration der Oberflächenschicht geeigneten Zellen auch andere Zellen und nichtzelluläre Gewebekomponenten aufweist, durch Zerkleinerung Gewebepartikel herzustellen, diese zu suspendieren und die Suspension durch einen vorgeformten, porösen künstlichen Träger zu filtrieren, und die folgenden Verfahrensschritte umfasst:
a) durch mechanische Zerkleinerung hergestellte Gewebepartikel mit Grössen von 0,5mm bis 4mm werden durch enzymatische Verdauung in einer Verdauungssuspension weiter zerkleinert und teilweise von nichtzellulären Gewebekomponenten befreit, wodurch die Gewebepartikel Dimensionen von unter 1mm erhalten,
b) die Verdauungssuspension wird durch Filtrieren von Partikeln befreit, welche Partikel durch ihre Grösse für einen Patienten ein Risiko bedeuten,
c) die aus der Filtration erhaltene Suspension von Gewebepartikeln wird ohne vorgängigen Zentrifugierschritt zur Auftrennung der Suspension durch den porösen Träger filtriert, wobei die Gewebepartikel im Bereich der Trägeroberfläche eingelagert werden und
d) der Träger wird gespült, dadurch, dass eine Spüllösung durch den porösen Träger gepresst wird in derselben Richtung in der in Verfahrensschritt c) filtriert wurde.

Die erfindungsgemässen Verbesserungen basieren im wesentlichen auf den folgenden Erkenntnissen:
- Auch wenn die Suspension aus einem Gewebe hergestellt wird, das neben den für die Einlagerung gewünschten Zellen grosse Mengen anderer Zellen und nichtzellulärer Gewebekomponenten aufweist, ist es nicht notwendig, die gewünschten Zellen aus dem Gemisch zu separieren (zu isolieren). Es genügt, das Gewebe genügend fein zu zerkleinern und zu suspendieren und den Träger sofort damit zu behandeln. Der Träger wird also nicht im hergebrachten Sinne mit einer Suspension von beispielsweise mikrovaskulären Zellen sondern mit einer Suspension von Partikeln des entnommenen Gewebes (bspw. Fettgewebe) behandelt.
- Die Gewebepartikel werden durch mechanische Zerkleinerung des entnommenen Gewebematerials und durch eine folgende enzymatische Verdauung hergestellt. Durch eine entsprechende mechanische Zerkleinerung wird sichergestellt, dass die entstehenden Gewebepartikel eine enge Grössenverteilung haben. Dadurch wird der Angriff beim enzymatischen Abbau regelmässiger und deren Produkt homogener.
- Sollte die Grössenverteilung der Gewebepartikel trotzdem den Anforderungen nicht genügen, beispielsweise dadurch, dass darin zu grosse Partikel für die Behandlung sehr kleinlumiger Gefässprothesen vorkommen, werden vor der Behandlung zu grosse Partikel durch Filtration der Suspension entfernt.
- Zusätzlich kann ein Grossteil des Fettanteiles durch Absetzenlassen und Trennen der Phasen aus der Suspension abgetrennt werden.
- Auch die aus der enzymatischen Verdauung entstehenden Nebenprodukte müssen nicht zwingend aus der Suspension entfernt werden, bevor der Träger damit behandelt wird. Der Träger wird nach der Einlagerung der Gewebepartikel, zur Reinigung mit einer Spüllösung gespült.

Im erfindungsgemässen Verfahren werden alle Teilschritte exakt kontrolliert und sind optimal aufeinander abgestimmt. Dies wurde möglich aufgrund neu entdeckter, unerwarteter Effekte, aufgrund derer der ganze Prozess unerwartet kurz, einfach und in einem geschlossenen System durchführbar wird. Diese Grundzüge der Erfindung und die damit verbundenen Vorteile sollen nun detailliert beschrieben werden.

Es hat sich überraschenderweise gezeigt, dass eine Trennung der durch die Verdauung erhaltenen Suspension durch Zentrifugation nicht notwendig ist. Die Zentrifugation kann, wenn notwendig, durch einfaches Absetzenlassen ersetzt werden. Die durch Absetzenlassen und Trennen der schwereren, die zu transplantierenden Zellen enthaltenden Schicht von der leichteren, fetthaltigen Schicht erzielte Reinigung ist bei der Bearbeitung von Fettgeweben ausreichend für die nachfolgende Behandlung des Trägers. Nach dem Auftragen der Suspension auf die Prothesen-Innenfläche können bei poröser Prothese mit einer Spülung durch die Prothesen-Wand weitere öligfettige Bestandteile ausgewaschen werden.

Lipid-Bestimmungen haben gezeigt, dass nur unmerklich höhere Fettmengen pro cm Prothesen-Länge zurück bleiben, wenn der Zentrifugations-Schritt durch eine Spülung direkt auf der Prothese ersetzt wird. In Übereinstimmung damit haben experimentelle Untersuchungen mit dem caninen Modell (Implantation in die A. carotis) den Beweis erbracht, dass Zellsuspensionen, die weitere Zellfraktionen enthalten, die nicht zur nativen Zellpopulation am Implantationsort gehören, zu keiner beobachtbaren Änderung im Heilprozess und in der Bildung der Neoarterie in der Prothese führen. Überraschenderweise war insbesodere keine Hypertrophie des Gewebes messbar und die rasterelektronenmikroskopischen Untersuchungen der inneren Prothesenoberfläche zeigten einen geschlossenen Endothelzellbelag.

Unter Berücksichtigung dieser beiden überraschenden Befunde, kann insbesondere auf die aufwendige Trennung nach Zelltypen verzichtet werden.

Darüber hinaus kann aber auch auf eine Reinigung der Zellsuspension zur Gewinnung der für die Geweberegeneration gewünschten Zellen überhaupt verzichtet werden, was erlaubt, die aus dem Verdauungs-Schritt gewonnene Zellsuspension direkt zur Beschichtung der Prothese zu verwenden. Das gilt insbesondere für den Fall von porösen Prothesen. Im caninen Modell hat sich nämlich ebenfalls überraschend gezeigt, dass eine poröse Prothese selbst als Filter benützt werden kann, das Zellen und Zellverbände zurückhält, während die gelösten Bestandteile, insbesondere auch die Enzyme für den Gewebeaufschluss, bspw. die Collagenase, ausgewaschen werden, so dass eine für die Implantation ausreichende Reinigung von Zellen und Zellverbänden erzielt wird. Der Reinigungsschritt durch Zentrifugation wird hier also durch eine Reinigung mittels Filtration auf der Prothese ersetzt. Diese Vereinfachung (keine Zentrifugation) erlaubt es, eine der Hauptanforderungen, nämlich jene bezüglich Sterilität, auf überraschend einfache Weise zu erfüllen. Zusätzlich wird der Prozess kontinuierlich und damit automatisierbar, was gemäss dem Stande der Technik nur mit einem aufwendigen Verfahren für kontinuierliche Zentrifugation möglich würde.

Überraschenderweise hat sich weiter im caninen Modell gezeigt, dass selbst die Abtrennung der Fettphase nach dem enzymatischen Gewebeaufschluss nicht erfoderlich ist. Das bedeutet, dass das Verfahren nochmals sehr vereinfacht werden kann, indem unmittelbar nach Aufschluss des Gewebes die gesamte Suspension mittels einer Pumpe über ein Stufenfilter (zur Abtrennung von zu grossen Gewebepartikeln) direkt auf die Prothese übertragen wird, welche die Zellen und Zellverbände zurückhält während alle gelösten Bestandteile durch eine nachfolgende Spülung sehr effizient und einfach ausgewaschen werden können.

Für die Beschichtung der Prothese sind Gewebepartikel notwendig, die möglichst vitale Zellen enthalten und deren Grösse derart ist, dass sie später in der mikroporösen Struktur des Trägermaterials eingelagert werden können, ohne zu stark in das Lumen der Prothese hineinzuragen und dadurch bei Freigabe des Blutstromes eventuell losgerissen zu werden, was zu Mikro-Embolien führen kann. Gewebepartikel mit einer möglichst uniformen Grösse und einem hohen Gehalt an vitalen Zellen werden erfindungsgemäss erhalten dadurch, dass der Verfahrensschritt der mechanischen Zerkleinerung und der Verfahrensschritt der enzymatischen Verdauung genau aufeinander abgestimmt werden.

Für den Aufschluss hat sich von vielen bekannten Enzymen, bspw. Pancreatin, Dispase, Trypsin und andere mehr, bei in-vitro-Versuchen Collagenase als besonders geeignet erwiesen. Durch ihre spezifische Wirkung trägt die Collagenase dazu bei, aus dem Gewebe einzelne Zellen, aber auch kleine Zellverbände herauszulösen, ohne die Zellen im wesentlichen in ihrer Vitalität zu schädigen. Hätte die Collagenase eine hundertprozentige Spezifizität für das Collagen der extrazellulären Matrix, könnte sie den Aufschluss des Gewebes eventuell bewirken, ohne die Zellen, respektive deren Oberfläche im geringsten zu schädigen. Dies ist insofern ein Idealbild, als sich gezeigt hat, dass Zellen, die über längere Zeit der Einwirkung von proteolytischen Enzymen, bspw. Collagenasen, ausgesetzt werden, Schädigungen an ihren Oberflächen-Rezeptoren aufweisen, und dass sie auch ihre Vitalität ganz verlieren können.

Das heisst mit anderen Worten, die Einwirkungszeit des Enzyms muss ausreichend lang sein, um das Gewebe in genügend kleine Partikel aufzuspalten, wobei die dafür notwendige Zeit sehr von der Grösse der zur Verfügung stehenden Teilchen abhängig ist. Sie darf aber auch nicht zu lange sein, um die Vitalität der herausgelösten Zellen nicht unnötig zu reduzieren. Da aus diesem Grunde für verschieden grosse Teilchen keine gemeinsamen optimalen Verdauungsparameter bestehen, muss durch eine entsprechende mechanische Zerkleinerung des Gewebes dafür gesorgt werden, dass die zu verdauenden Gewebeteile möglichst klein und möglichst alle gleich gross sind.

Erfindungsgemäss geht der enzymatischen Verdauung eine derartige zellschonende, mechanische Zerkleinerung voraus, dass dadurch Gewebeteile entstehen, die möglichst alle gleich gross sind. Zerkleinerungsverfahren sind zellschonend (atraumatisch) wenn das Gewebe geschnitten und nicht gequetscht oder zerschlagen wird. Dies wird erfindungsgemäss erreicht durch die Behandlung des Gewebes mit einer Mehrzahl von sehr scharfen, koordiniert bewegten Klingen.

Eine enge, genau definierte Grössenverteilung der zu verdauenden Gewebeteilchen erlaubt es, eine definierte und optimierte Einwirkungszeit der Collagenase zu bestimmen.

Da es sich bei diesem Verfahren zur Herstellung von Gefässprothesen um eine Gewebetransplantation handelt, bei der vitale Zellen enthaltende Gewebepartikel auf einem Träger so implantiert werden, dass sich das entsprechende Organ, in diesem Fall bspw. eine Gefässprothese oder eine Herzklappe, wieder entsprechend der vorgegebenen Form des Gerüsts neu bilden können, so ist es denkbar, dass dieses Prinzip der Gewebetransplantation auch für andere Organe, bspw. Pancreas oder Milz anwendbar ist, wenn der Prozess in entsprechender Weise an die notwendigen Zelltypen und Gewebe angepasst wird.

### Ausführungsbeispiel

Ein Ausführungsbeispiel des erfindungsgemässen Verfahrens wird nun am Beispiel einer endothelialisierten Gefässprothese eingehend diskutiert Der wesentliche Ablauf des Verfahrens besteht aus:
1. der Herstellung einer Suspension von Gewebepartikeln aus einem körpereigenen Gewebematerial,
2. einer Abtrennung von zu grossen Gewebeteilchen und/oder eines Grossteils der Fettanteile,
3. einer (allfälligen) Zwischenlagerung der Suspension,
4. der Behandlung des vorgeformten känstlichen Trägers mit der Suspension,
5. einer Spülung der Prothese,
6. einer (allfälligen) Behandlung der Prothese zur Erhaltung der für die implatierten Zellen notwendigen Feuchtigkeit,
7. einer (allfälligen) Zwischenlagerung der hergestellten Prothese.

Dabei sind für den Humanfall die Schritte 1, 2, 4 und 5 notwendig, die übrigen Schritte können zur Anwendung kommen oder weggelassen werden, je nach Art der Prothese und Durchführung der Operation.

Das entnommene Gewebe, beispielsweise, Omentum, Granulationsgewebe (tissue in sponge matrix gemäss Bishop et al., Vascular endothelial differentiation in sponge matrix allografts, Hum.Immunol., 28:128-133, 1990) oder ein anderes gefässreiches Gewebe wird, um die Traumatisierung der Zellen so gering wie möglich zu halten, sehr schonend mechanisch zerkleinert, indem es mit einer Mehrzahl sehr scharfer, koordiniert bewegter Klingen behandelt wird. Das Ziel ist, einen möglichst hohen Prozentsatz vitaler Zellen bei gleichzeitig hoher absoluter Zellausbeute zu erhalten. Trotz diesen Anforderungen soll die Zerkleinerung in relativ kurzer Zeit erfolgen, bspw. 1-3 Minuten/100 g Gewebemasse.

Die Grösse der Gewebeteile, die mechanisch zerkleinert werden, ist durch die Dimension des zu verwendenden Zerkleinerungs-Gerätes begrenzt; typische Eingangsgrössen sind Stücke mit Abmessungen von 3x3x2 cm, 5x5x2 cm oder, in Gewicht ausgedrückt, Stücke von 20g bis etwa 50g. Diese Stücke sollen auf 0,5mm-4mm, vorzugsweise 0,5mm-2mm in Scheiben, Stäbchen oder Würfel zerkleinert werden. Bei Gewebewürfeln der Grösse von 3x3x3 mm bis 4x5x5mm entspricht das einem Gewicht von ca. 0,01g bis 0,1g.

Anschliessend wird das mechanisch zerkleinerte Gewebe enzymatisch aufgeschlossen, bspw. mit Collagenase, um die Zellen wenigstens teilweise aus dem Bindegewebe zu befreien und dadurch die Gewebepartikel weiter zu zerkleinern. Dabei werden die kollagenen und elastischen Anteile des Bindegewebes abgebaut, so dass die Zellen freigesetzt, aber möglichst wenig geschädigt werden (minimaler Abbau von Bestandteilen der Zelloberfläche). Auch der enzymatische Abbau soll möglichst rasch (aber schonend) erfolgen, was über die Temperatur und die Enzymkonzentration gesteuert wird. Ein Bewegen des Ansatzes bewirkt eine gute Durchmischung der Phasen, was den Abbau zusätzlich beschleunigt.

Verschiedene solche Vorgehen zur Bearbeitung von lebenden Zellen sind bekannt und können entsprechend angewendet werden. Überraschenderweise hat es sich gezeigt, dass das Rühren mit einem Magnetrührer effizienter ist als ein Schütteln des ganzen Reaktionsgefässes. Als Magnetrührstäbe eignen sich Körper ohne scharfe Kanten, vorzugsweise abgerundete Stäbchen, da mit solchen ein Aufwickeln des Gewebes verhindert wird. Der Rührer ist freischwebend oder hat nur minimalen Bodenkontakt, womit auch ein Zermahlen des suspendierten Zellmaterials verhindert wird.

Für den Verdauungsschritt kann bspw. Collagenase verwendet werden, eine empfohlene Konzentration ist ca. 400 bis 20'000 Mandl-U/ml, typischerweise 750-3'000 U/ml, die Temperatur mindestens 37°C aber nicht über 40°C, vorzugsweise 37-38°C und das Volumenverhältnis von Gewebe und Enzymlösung 1:1 bis 1:2.

Nach dem Verdauungsschritt kann die Suspension für eine partielle Reinigung aufgetrennt werden. Auch dieser Teil des Prozesses muss schnell durchführbar sein, da man es einerseits mit Material zu tun hat, dessen Stoffwechselaktivität bei den Verarbeitungstemperaturen relativ hoch ist, im Gegensatz zur Verarbeitung von gekühlten Gewebematerialien, und andererseits ein rasch durchführbares Verfahren aus medizinischen Gründen angestrebt wird.

Eine Reinigung der Suspension mit dem Ziele der möglichst quantitativen Abtrennung der verwendeten Enzyme oder der Abtrennung von anderen als Endothelzellen ist, wie bereits erwähnt, nicht notwendig. Es hat sich gezeigt, dass keine Nachteile, wie bspw. vermehrte Hypertrophie der neuen Gewebeschicht, entstehen, wenn diese Reinigung weggelassen oder sehr vereinfacht, das heisst nur mit einem partiellen Reinigungsresultat durchgeführt wird. Das heisst mit anderen Worten, es entstehen keine bekannten Nachteile, wenn bspw. nicht eine reine Endothelzellsuspension, sondern das Gemisch aller aus dem Fettgewebe isolierten Zellen zur Transplantation verwendet wird.

Eine teilweise Trennung der Suspension zur Entfernung von zu grossen Partikeln oder eines Teiles der fetthaltigen Anteile kann hingegen von Vorteil sein. Diese kann entweder unter Ausnutzung des spezifischen Gewichtes (Absetztenlassen und Abtrennen der leichteren Phase) und/oder der Grösse der Aggregate (Filtration) durchgeführt werden. Die Trennung nach Grösse ist vor allem bei der Verwendung von kleinlumigen Prothesen wichtig, um eine Verengung des Lumens durch zu grosse transplantierte Teilchen zu verhindern. Aber auch bei grosslumigen Prothesen ist eine Trennung nach Grösse vorteilhaft, denn für grosse aufgetragene Teilchen ist das Risiko, dass sie wieder von der Oberfläche abgelöst werden und als Mikrothromben in der Endstrombahn zu Problemen führen, gross. Die Trennung nach spezifischem Gewicht ist bei anderen Anwendungen vorteilhaft. Beide Trennmethoden werden nachfolgend kurz besprochen.

Zuerst die Trennung nach spezifischem Gewicht (Absetzenlassen und Trennen der Schichten): Bei Fettgeweben flottieren die Teilchen der Suspension, die einen genügend grossen Fettanteil haben, relativ rasch nach oben. Entgegen der herrschenden Meinung ist eine forcierte Sedimentation durch Zentrifugieren nicht nötig, sondern reicht eine Sedimentation im Gravitationsfeld. Das bringt den Vorteil, dass die Sedimentation in einem geschlossenen und ruhenden System durchgeführt werden kann.

Man kann die Sedimentation beschleunigen, bspw. durch die geeignete Wahl der Tiefe des Sedimentationsgefässes, durch Verdünnung der Suspension nach dem Abschluss des enzymatischen Aufschlusses und/oder durch andere geeignete Massnahmen. Beispiel: die zu sedimentierende Suspension wird mit Pufferlösung auf das 1,5 bis 10fache, vorzugsweise auf das 1,5- bis 2-fache des Volumens verdünnt, bspw. 300 g Gewebe + 300 ml Enzymlösung = Suspension für die Verdauung (Aufschluss), welche für die Sedimentation auf 1000 ml aufgefällt wird (was einer 1,67fachen Verdünnung entspricht). In einem zylindrischen Gefäss von 15 cm Höhe und 10 cm Durchmesser dauert die Sedimentation für diese Volumina 1 - 15 Minuten, vorzugsweise 3 - 10 Minuten. Nach dieser Zeit zeigt sich eine deutliche Grenze zwischen wässriger und öliger Phase. Man kann die Sedimentation durch sehr langsames Rühren mit einem Magnetrührer noch unterstützen, da dadurch im Sediment eingeschlossene, leichtere Bestandteile befreit werden und da, vor allem bei dichten Suspensionen (Collagenase : Gewebe = 1:1), die Trennung der Gewebepartikel nach ihrem spezifischen Gewicht durch die zusätzliche, schwache Bewegung gefördert wird.

Sedimentation und Trennung der dadurch entstandenen Schichten wird vorzugsweise unmittelbar anschliessend an die enzymatische Verdauung, vorzugsweise im gleichen Gefäss und bei 20 - 40 °C, für Fettgewebe vorzugsweise bei 30 - 37 °C durchgeführt.

Für die Beschichtung von Gefässprothesen für die Humanmedizin ist eine Abtrennung zu grosser Partikel notwendig. So sollen bspw. bei Gefässprothesen mit einem Innendurchmesser von 4mm jene Teilchen ausgeschlossen werden, deren Durchmesser grösser als 0,4mm und deren Länge grösser als 1mm ist. Dazu sind Siebe mit einer Maschenweite von 0,2mm bis 1mm, vorzugsweise 0,2mm bis 0,4mm nötig. Um die Zellausbeute zu verbessern und um Verstopfungen zu verhindern, sind bei grösseren Materialmengen (ab 50g Gewebe) mehrstufige Siebe empfehlenswert. Bis zu 300g Gewebe bspw. 3 Stufen, wobei der Durchmesser der Siebe 4cm - 6cm und der Abstand zwischen zwei Sieben 2cm bis 6cm beträgt. Die Maschenweite der Siebe wird bspw. wie folgt gewählt: ca. 2mm für das erste, ca. 0,8mm für das zweite und ca. 0,25mm für das dritte Sieb. Damit wird vermieden, dass die ganze Masse der zurückbleibenden Fraktion eine einzige Siebschicht mehr und mehr belädt, wodurch sich ein zunehmender Strömungswiderstand bildet, und aber vor allem auch der Siebeffekt drastisch verändert wird, so dass mit zunehmender Beladung die Grösse der passierenden Teilchen abnimmt, wodurch erhebliche Verluste an Zellmaterial auftreten. Bei mehrstufigen Sieben wird dagegen die Gesamtmenge der Anzahl und der Maschenweite der eingesetzten Siebe entsprechend verteilt und der Strömungswiderstand und der Siebeffekt viel weniger beeinflusst.

Die Siebe werden vorzugsweise horizontal übereinander angeordnet, um die Filtration mittels Schwerkraft durchführen zu können, was eine gleichmässigere Siebbeladung und damit wiederum eine definiertere Filtration ermöglicht. Ferner werden bei einer solchen Anordnung weniger Geräte benötigt, was auch den strikten Anforderungen an die Sterilität wiederum entgegenkommt.

Es sind aber auch Filtrationen mittels Pumpentransport möglich, bei welchen im wesentlichen die gleichen Kriterien beachtet werden müssen. Ein Pumpentransport hat aber vor allem den Vorteil, dass damit eine Filtration entgegen der Schwerkraft durchgeführt werden kann, die einen luftblasenfeien Flüssigkeitstransport auf einfache Weise ermöglicht, was für die nachfolgende Behandlung des Trägermaterials wichtig ist.

Als Pumpen werden Schlauchquetschpumpen eingesetzt, vorzugsweise solche mit 2-Rollen-Köpfen. Es hat sich überraschenderweise gezeigt, dass damit nur unerhebliche Verluste an vitalen Zellen entstehen. Dagegen ermöglicht die Anwendung solcher Pumpen auf einfache Weise einen kontrollierten, nicht einfach der Schwerkraft unterworfenen Prozess in einem geschlossenen System, was nicht nur klinisch bedeutsam ist bezüglich der Sterilität, sondern auch für die zeitlich kontrollierte Durchführung des Verfahrens.

Die Kombination von Zellreinigung nach spezifischem Gewicht und nach Grösse ist dann angezeigt, wenn sowohl Fett als auch andere Partikel über einer gegebenen Grösse ausgeschlossen werden müssen, bspw. für kleinlumige Gefässprothesen.

Die Endothelzellen für die Herstellung der Prothesen befinden sich vorwiegend in der wässrigen Phase (Trennung nach spezifischen Gewicht). Die wässrige Phase wird vorzugsweise von der öligfettigen Phase dadurch abgetrennt, dass die wässrige Phase durch die Oeffnung am oder nahe am Boden des Gefässes für die Sedimentation entnommen wird, so dass die öligfettige Phase im Verdauungs und/oder Sedimentationsgefäss zurückbleibt.

Gelöste Komponenten der Suspension, wie die Collagenase, die für den enzymatischen Abbau verwendet werden, und flüssige Komponenten, wie öliges Fett aus dem Gewebe, vor allem, falls nur eine Trennung nach der Grösse durchgeführt wurde, werden erst später bei/nach der Behandlung des Trägermaterials entfernt.

Um den hohen Anforderungen an die Sterilität des Verfahrens zu genügen, muss die Suspension während des ganzen Prozesses, vorzugsweise einschliesslich der Auftragung auf das Trägermaterial in einem geschlossenen System gehalten werden, das auch den Träger enthält. Das wird ermöglicht durch bspw. Schlauchverbindungen zwischen dem Verdauungsgefäss und dem Beschichtungsmodul, mit welchem das Einlagern des Zellmaterials in der Oberfläche des Trägermaterials (bei Gefässprothesen bspw. auf die innere Fläche) durchgeführt wird. Der Flüssigkeitstransport kann mittels Schwerkraft erfolgen (verschiedene Höhen bei der Anordnung der Geräte) oder mittels Schlauchquetschpumpen, Rollerpumpen oder andere Verdrängungspumpen, vorzugsweise solchen, bei denen die Suspension nur mit Einwegteilen in Kontakt kommt. Im folgenden Beispiel wurde für die Flüssigkeitsförderung zum Auftragen des Zellmaterials auf ein Prothesenmaterial eine Watson-Pumpe mit 2 Rollen mit einem Silikonschlauch von ca. 8mm Durchmesser und ohne Schlupf verwendet. Dabei hat sich gezeigt, dass die Anzahl vitaler Zellen in der Suspension auch durch fünfmaliges Pumpen nicht signifikant verringert wird.

Für die Einlagerung des Zellmaterials werden je nach Anforderung zwei wählbare Methoden angewendet. Ist das Trägermaterial durchlässig porös, so wird die Filtrationstechnik angewendet, ist das Prothesenmaterial nicht oder zu wenig durchlässig oder kann aus irgendeinem Grund die Filtrationstechnik nicht angewendet werden, so wird die Rotationstechnik angewendet, bei der das Zellmaterial mittels Beschleunigung (Zentrifugation) auf die Oberfläche des Prothesenmaterials aufgetragen wird (Publikation EP-462051).

Der Vorteil bei der Filtration der Zell-Suspension durch ein poröses Material liegt darin, dass in einem Arbeitsgang zwei Schritte erfolgen, nämlich die Einlagerung der Gewebepartikel in die Oberfläche der Prothese, sowie das Auswaschen von gelösten Substanzen, wie die Collagenase, und/oder von flüssigen Phasen der Suspension, wie öliges Fett. Auf diese Weise wird das Zellmaterial im gleichen Arbeitsgang nachgereinigt (zweite partielle Reinigung).

Wichtig ist der Grad der Porosität des Prothesenmaterials bzw. der Prothese, er wird bspw. anhand der Wasserdurchlässigkeit bestimmt (in diesem Fall mit der Wesolewski-Methode gemessen). Prothesen mit höherer Dichte (weniger als 100 ml Wasserdurchlass pro cm² pro Minute) erfahren beim Auftragen des Zellmaterials eine andere Behandlung als Prothesen mit einer geringeren Dichte (über 100ml/cm²min). Beide Materialien lassen auf jeden Fall, zumindest in der Anfangsphase des Auftragens, Flüssigkeit in das Material ein- und durchtreten. Untersucht man anschliessen die erzielte Zellablagerung auf der Oberfläche der Prothese (hier die Rohrinnenwand), so kann man feststellen, dass sich die meisten Zellen nahe der inneren Oberfläche befinden, was ja auch erwünscht ist. Beispielsweise bei einer Wandstärke von 0,2mm und einer annähernd gleichmässigen Verteilung von Fibrillen aus Dacron® sind die mehrzelligen Aggregate vorwiegend in den obersten 5µm lokalisiert, während Einzelzellen in abnehmender Anzahl bis in etwa 100µm Tiefe auftreten. Bei grösseren Poren werden diese mit entsprechend grossen Aggregaten gefüllt oder beladen.

Auf diese Weise wird auch ein Aufschwimmen der Zellen, die auf oder nahe der Oberfläche abgelagert wurden, durch die Rauheit der Prothesenoberfläche erschwert oder fast ganz verunmöglicht. Strömungsexperimente mit derart beschichteten Prothesen - Durchströmung in Längsrichtung mit physiologischer Salzlösung bei Geschwindigkteiten wie in peripheren Arterien - zeigten nämlich überraschenderweise, dass schon unmittelbar nach der Filtration bei physiologischen Blutstromgeschwindikgeiten kaum Zellen ausgewaschen werden, während die biologisch aktive Adhärenz auf glatten Oberflächen nach 60 Minuten erst etwa zu 60% abgeschlossen ist (Jarrell-Radomski et al., Immediate vascular graft monolayers using microvessel endothelial cells, in: Endothelial Seeding in Vascular Surgery, edited by Herring, M. and Clover, J.L. Grüne & Stratton Inc. 1987, S. 37-55).

Um eine Ablagerung der Zellen in genügender Tiefe zu erreichen, wird für ein dichteres (feinerporiges) Prothesematerial mehr Druck zum Auftragen nötig sein, als bei einem lockerer gepackten Material. Dagegen muss bei einem solchen Material wiederum die Fördermenge ausreichend sein, um den schnellen Flüssigkeitsschwund während des Auftragens zu kompensieren. In beiden Fällen muss, abgesehen von kapillaren Wirkungen, der Druckgradient normal zur beschickten Oberfläche entsprechend eingestellt werden.

Bei der Rotationstechnik (Publikation EP-462051) eignet sich vor allem bei sehr dichten Prothesen, welche praktisch keine für die Filtrationsmethode brauchbare Porosität aufweisen.

Um das Risiko von Embolien durch Ablösen von aufgetragenen Zellen und Zellverbänden sowie anderen kleinen Gewebeteilchen zu reduzieren, kann die Gefässprothese anschliessend an eine Spülung durch die Gefässwand (radiale Spülung) auch noch durch das Lumen der Prothese (axiale Spülung) gespült werden. Man pumpt zu diesem Zweck mit angenähert physiologischer Geschwindigkeit (Blutgeschwindigkeit am Implantationsort) bspw. 200ml bis 400ml physiologische Lösung pro Minute in axialer Richtung durch die Prothese. Nach Zellauftrag durch Filtration können dabei i.a. nur sehr wenig Zellen und wenig anderes Gewebematerial ausgewaschen werden, was die Mikroembolie-Sicherheit des Verfahrens demonstriert.

Damit die Zellvitalität der mit Zellmaterial behandelten Prothesen bis zur Anwendung erhalten bleibt, können diese als letzter Schritt des Verfahrens bis zum Wundverschluss noch speziell geschützt werden. Die Zellschicht der vorpräparierten Prothesenoberfläche wird dadurch vor allem vor dem Eintrocknen geschützt.

Das hier diskutierte Verfahren zur Herstellung von Auto-Transplantaten gemäss Erfindung ist für Transplantate insbesondere im Gebiet der Humanmedizin aber auch der Veterinärmedizin gleichermassen anwendbar. Im Gegensatz zu einer üblichen Transplantation, bei der Organe oder Organteile transplantiert werden (Organtransplantation), sind es hier Zellen und/oder Zellverbände, die auf einem biokompatiblen Träger transplantiert werden (Zell-Transplantation) und die notwendig sind, damit es nach dem Einsetzen der Prothese im Körper zum Neuaufbau eines entsprechenden Organs, bspw. einer Neoarterie, kommen kann.

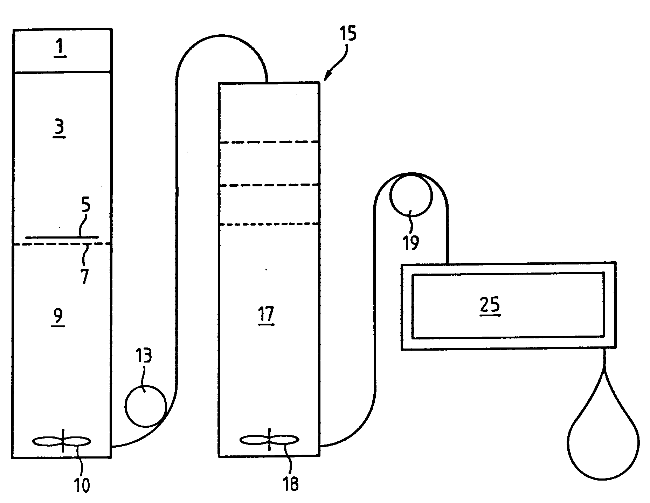

Anschliessend wird nun unter Zuhilfenahme der Figur ein skizzenhaftes Beispiel einer geschlossenen Einrichtung zur Durchführung des erfindugnsgemässen Verfahrens diskutiert. Bei den einzelnen Gerätekomponenten handelt es sich teilweise um solche, die auf dem Markt erhältlich sind. Die Zusammenschaltung dieser Geräte entsprechend dem hier beschriebenen neuen, komprimierten und überraschenderweise biologisch sehr wirksamen Verfahren entspricht hingegen dem erfindungsgemässen Verfahrensablauf und stellt somit eine Einrichtung zur Herstellung von Prothesen gemäss Patentanspruch 1 dar. Die Einrichtung kann derart gestaltet sein, dass sie auch in einem Operationssaal verwendet werden kann und die Herstellung von erfindungsgemässen Prothesen vor Ort ermöglicht.

Die Figur zeigt in schematisch vereinfachter Weise eine vollständig abschliessbare Einrichtung, in welcher das ganze Verfahren in einem zusammenhängenden geschlossenen Gefäss- und Schlauchsystem durchgeführt werden kann. Sie lässt sich leicht an einem fahrbaren Gestell anordnen und ist auf diese Weise mobil für den Einsatz beispielsweise im Labor oder im Operationssaal. Die bildliche Geschlossenheit schliesst natürlich die Zuführungen von zu verarbeitendem Gewebe, Reagentien und Lösungen nicht aus. Doch soll durch die Geschlossenheit gezeigt werden, wie man den sehr strengen Sterilitätsanforderungen gerecht werden kann.

Eine erste Einheit umfasst die Geräte für die mechanische Zerkleinerung, einen Antrieb 1, eine Förderstrecke 3, die auf ein Messer/Lochplattenpaar 5,7 gerichtet ist, und für den Aufschluss des Gewebematerials zur Freisetzung der Zellen eine Verdaungszelle 9 mit einem Rührer 10 zur Unterstützung des Aufschlusses und einer ersten Sedimentation. Die Geräte können so geschaltet sein, dass die zerkleinerte Gewebemasse von der Lochplatte gleich in das Verdauungsgefäss fällt. Nach Abschluss der Verdauung kann in diesem Gefäss auch die Sedimentation stattfinden, um die öligfettige Phase abzutrennen. Dazu kann zu Beginn der Sedimentation das Gefäss mit Puffer weiter aufgefüllt werden, um die Trennung zu unterstützen. Ferner kann mit demselben Ziel, beispielsweise mit einem Magnetrührer, eine leichte Bewegung von 0,1-1 U/sec erzeugt werden. Die in der ersten Einheit entstandene Suspension wird durch ein Fördermittel 13 in eine zweite Einheit überführt, die aus einem Filterteil 15 und einem Auffanggefäss 17 besteht. Während der Förderung in das Gefäss 17 wird im Gefäss 9 ebenfalls eine leichte Strömung erzeugt. Der Filterteil ist hier ein Mehrstufenfilter zur Gewinnung von Partikeln bestimmter Grösse, welche als Suspension im Auffanggefäss gesammelt werden. In dieser Form ist die Sedimentationsneigung der Suspension nur noch gering und wird im Gefäss 17 durch einen zweiten Magnetrührer 18 aufgehoben. Ein weiteres Fördermittel 19 überführt die nun fertig einsetzbare, homogene Suspension in die dritte Einheit 25 zur Prothesenbeschichtung. Diese Einheit besteht vorzugsweise aus einer Handschuhbox, in welcher die zur Beschichtung nötigen Geräte untergebracht sind und die unbehandelten Prothesen zur Beschichtung schon bereitliegen. In dieser Box 25 findet die Konditionierung der trocken gelagerten Prothese mit physiologischer Lösung statt, unmittelbar bevor die Zellen auf die Prothesen aufgetragen werden. Das Auftragen/Beschichten kann von Hand, aber auch maschinell durchgeführt werden. Die fertigen Prothesen können, wahlweise auch zusätzlich mit einem Schutz gegen Austrocknung versehen und adäquat verpackt, mittels einer Schleuse dem sterilen Inneren der Beschichtungsbox entnommen und mit Hilfe weiterer spezieller Einrichtungen auf den sterilen Operationstisch sicher übergeben werden. Je nach Grösse und Aufwand der Gesamt-Einrichtung kann sie für die serienweise Herstellung von Prothesen oder für die ad hoc Herstellung von einzelnen Prothesen im Operationssaal ausgerüstet sein.

Der Filter 15 kann auch ohne Gefäss 17 zwischen Gefäss 9 und Beschichtungseinheit geschaltet werden, derart, dass die Suspension vom Filter ohne Zwischenlagerung direkt auf die Prothese geführt wird.

## Patentansprüche

1. Verfahren zur Herstellung von insbesondere in der Humanmedizin anwendbaren Endoprothesen, welche Berührungsflächen für den direkten Kontakt mit Blut und/oder Lymphe und an diesen Berührungsflächen angelagerte, eine Regeneration einer lebenden Oberflächenschicht initiierende Zellen und Zellverbände aufweisen, wobei das Verfahren daraus besteht, aus körpereigenem Gewebe, das neben den für die Regeneration der Oberflächenschicht geeigneten Zellen auch andere Zellen und nichtzelluläre Gewebekomponenten aufweist, durch Zerkleinerung Gewebepartikel herzustellen, diese zu suspendieren und die Suspension durch einen vorgeformten, porösen künstlichen Träger zu filtrieren, **gekennzeichnet**, durch die folgenden Verfahrensschritte:
a) durch mechanische Zerkleinerung hergestellte Gewebepartikel mit Grössen von 0,5mm - 4mm werden durch enzymatische Verdauung in einer Verdauungssuspension weiter zerkleinert und teilweise von nichtzellulären Gewebekomponenten befreit, wodurch die Gewebepartikel Dimensionen von unter 1mm erhalten,
b) die Verdauungssuspension wird durch Filtrieren von Partikeln befreit, welche Partikel durch ihre Grösse für einen Patienten ein Risiko bedeuten,
c) die aus der Filtration erhaltene Suspension von Gewebepartikeln wird ohne vorgängigen Zentrifugierschritt zur Auftrennung der Suspension durch den porösen Träger filtriert, wobei die Gewebepartikel im Bereich der Trägeroberfläche eingelagert werden und
d) der Träger wird gespült, dadurch, dass eine Spüllösung durch den porösen Träger gepresst wird in derselben Richtung in der in Verfahrensschritt c) filtriert wurde.

2. Verfahren nach Anspruch 1, **dadurch gekennzeichnet**, dass als körpereigenes Gewebe Omentum oder subkutanes Fettgewebe verwendet wird.

3. Verfahren nach einem der Ansprüche 1 oder 2, **dadurch gekennzeichnet**, dass die mechanische Zerkleinerung durch eine Mehrzahl korreliert bewegter, scharfer Klingen realisiert wird.

4. Verfahren nach einem der Ansprüche 1 bis 3, **dadurch gekennzeichnet**, dass durch Absetzenlassen der im Verfahrensschritt b) oder c) erhaltenen Suspension und durch Weiterverwendung der schwereren Schicht Fettbestandteile aus der Suspension abgetrennt werden.

5. Verfahren nach einem der Ansprüche 1 bis 4, **dadurch gekennzeichnet**, dass für den Verfahrensschritt d) die Schwerkraft oder eine durch Rotation des Trägers erzeugte Zentrifugalkraft ausgenützt wird.

6. Verfahren nach einem der Ansprüche 1 bis 5, **dadurch gekennzeichnet**, dass zur Entfernung von ungenügend fixierten Gewebepartikeln der Träger nach dem Verfahrensschritt d) mit einem parallel zur behandelten Oberfläche geführten Spüllösungsstrom behandelt wird.

7. Einrichtung zur Durchführung des Verfahrens nach Anspruch 1, **dadurch gekennzeichnet**, dass sie ein mit einem Rührmittel (10) versehenes Gefäss (9) für die enzymatische Verdauung von Gewebepartikeln aufweist mit einem Eingang, in dessen Bereich ein Mittel (1/3/5/7) zur mechanischen Zerkleinerung von Gewebe zu Gewebepartikeln angeordnet ist, und mit einem Ausgang, der in einem in Richtung Schwerkraft unteren Bereich des Gefässes (9) angeordnet ist, dass sie Mittel (25) zum Filtrieren einer Suspension von Gewebepartikeln durch einen vorgeformten, porösen künstlichen Träger und zum Spülen des Trägers in derselben Richtung aufweist, wobei der Ausgang des Gefässes (9) und die Mittel (25) zum Filtrieren durch eine Leitung miteinander verbunden sind, durch welche Leitung eine Suspension von Gewebepartikeln förderbar ist und wobei zwischen dem Gefäss (9) zur enzymatischen Verdauung und Mittel (25) zur Filtrierung eine Filtereinheit (15) zum Zurückhalten von Partikeln, die für einen Patienten durch ihre Grösse ein Risiko bedeuten, vorgesehen ist, wobei die Einrichtung ein abschliessbares System ohne Zentrifugiermittel zur Auftrennung der Verdauungssuspension zwischen dem Gefäss (9) zur enzymatischen Verdauung und Mittel (25) zur Filtrierung darstellt.

8. Einrichtung nach Anspruch 7, **dadurch gekennzeichnet**, dass die Filtereinheit (15) in einem weiteren Gefäss (17) mit einem Rührmittel (18) angeordnet ist.

9. Einrichtung nach einem der Ansprüche 7 oder 8, **dadurch gekennzeichnet**, dass die Rührmittel (10, 18) Magnetrührer sind.

10. Einrichtung nach einem der Ansprüche 7 bis 9, **dadurch gekennzeichnet**, dass die Leitungen zwischen Gefäss (9) und Mittel (25) zur Filtration Einwegsehläuche sind und dass zur Förderung der Suspension Quetschpumpen (13, 19) vorgesehen sind.

11. Verwendung von Verfahren und Einrichtung nach einem der Ansprüche 1 bis 10 zur Herstellung von Gefässprothesen.

12. Verwendung von Verfahren und Einrichtung nach einem der Ansprüche 1 bis 10 zur Herstellung von Herzklappen.

## Claims

1. Process for the production of endoprostheses, more particularly usable in human medicine, which have contact surfaces for direct contact with the blood and/or lymph and cells and cell bonds or unions initiating a regeneration of a living surface layer deposited on said contact surfaces, the process also consisting of preparing tissue particles by comminution from endogenic tissue which, apart from the cells suitable for regenerating the surface layer, also has other cells and non-cellular tissue components, which are suspended and the suspension is filtered through a preshaped, porous artificial support, characterized by the following process steps:
a) tissue particles with sizes of 0.5 mm to 4 mm produced by mechanical comminution are further comminuted by enzymatic digestion in a digestion suspension and partly freed from non-cellular tissue components, the tissue particles being given dimensions below 1 mm,
b) the digestion suspension is freed by filtration from particles, which represent a risk for a patient due to their size,
c) the tissue particle suspension obtained by filtration is filtered through the porous support without any preceding centrifuging step for separating the suspension, the tissue particles being incorporated in the area of the support surface and
d) the support is rinsed in that a rinsing solution is pressed through the porous support in the same direction in which process step c) was filtered.

2. Process according to claim 1, characterized in that as the endogenic tissue omentum or subcutaneous adipose tissue is used.

3. Process according to one of the claims 1 or 2, characterized in that mechanical comminution is performed by a plurality of sharp blades moved in a correlated manner.

4. Process according to one of the claims 1 to 3, characterized in that by allowing the suspension obtained in process steps b) or c) to settle and by further use of the heavier layer fatty constituents are separated from the suspension.

5. Process according to one of the claims 1 to 4, characterized in that for process step d) use is made of gravity or a centrifugal force produced by rotation of the support.

6. Process according to one of the claims 1 to 5, characterized in that for removing inadequately fixed tissue particles the support of process step d) is treated with a rinsing solution flow guided in parallel to the treated surface.

7. Apparatus for performing the process according to claim 1, characterized in that it has a vessel (9) provided with a stirrer (10) for the enzymatic digestion of tissue particles and having an inlet, in whose vicinity is located a means (1/3/5/7) for the mechanical comminution of tissue to tissue particles, and having an outlet, which is located in a lower area of the vessel (9) in the gravity direction, that means (25) are provided for filtering a suspension of tissue particles through a preshaped, porous, artificial support and for rinsing the support in the same direction, the outlet of the vessel (9) and the filtering means (25) being interconnected by a line through which a tissue particle suspension can be fed and in which between the enzymatic digestion vessel (9) and the filtering means (25) is provided a filter unit (15) for retaining particles, whose size would represent a risk for a patient, the apparatus constituting a sealable system without centrifuging means for the separation of the digestion suspension between the enzymatic digestion vessel (9) and filtering means (25).

8. Apparatus according to claim 7, characterized in that the filter unit (15) is placed in a further vessel (17) with stirring means (18).

9. Apparatus according to one of the claims 7 or 8, characterized in that the stirring means (10, 18) are magnetic stirrers.

10. Apparatus according to one of the claims 7 to 9, characterized in that the lines between the vessel (9) and filtering means (25) are disposable hoses and hose compression pumps (13, 19) are provided for delivering the suspension.

11. Use of the process and apparatus according to claims 1 to 10 for producing vascular prostheses.

12. Use of the process and apparatus according to one of the claims 1 to 10 for producing cardiac valves.

## Revendications

1. Procédé d'obtention d'endoprothèses utilisables en particulier en médecine humaine, qui présentent des surfaces de contact pour le contact direct avec le sang et/ou la lymphe et des cellules et groupes de cellules déposés sur ces surfaces de contact et initiant la régénération d'une couche de surface vivante, le procédé résidant dans le fait que des particules tissulaires sont produites par hachage à partir de tissu autogène qui présente, outre les cellules propres à régénérer la couche de surface, d'autres cellules et composants tissulaires non cellulaires, que ces particules sont mises en suspension et la suspension est filtrée à travers un support artificiel poreux préformé, caractérisé en ce qu'il comporte les étapes suivantes :
a) des particules de tissu obtenues par hachage mécanique, d'une grosseur de 0,5 à 4 mm, sont encore réduites par digestion enzymatique dans une suspension de digestion et partiellement dégagées des composants non cellulaires du tissu, les particules de tissu prenant une dimension de moins de 1 mm ;
b) la suspension digérée est débarrassée par filtration des particules dont la taille crée un risque pour le patient ;
c) la suspension de particules tissulaires obtenue par filtration est filtrée à travers le support poreux sans étape de centrifugation préalable pour la décomposition de la suspension, les particules de tissu se déposant dans la région de la surface du support, et
d) le support est rincé par l'injection sous pression d'une solution de rinçage à travers le support poreux, dans le même sens que la filtration a eu lieu dans l'étape c).

2. Procédé selon la revendication 1, caractérisé en ce que l'on utilise comme tissu autogène l'épiploon ou du tissu adipeux sous-cutané.

3. Procédé selon l'une quelconque des revendications 1 ou 2, caractérisé en ce que le hachage mécanique est réalisé par une pluralité de lames affûtées déplacées de façon corrélée.

4. Procédé selon l'une ou l'ensemble des revendications 1 à 3, caractérisé en ce que la décantation de la suspension obtenue dans l'étape b) ou c) du procédé et l'utilisation de la couche la plus lourde permet de séparer les composants adipeux de la suspension.

5. Procédé selon l'une ou l'ensemble des revendications 1 à 4, caractérisé en ce que la gravité ou une force centrifuge obtenue par rotation du support est exploitée dans l'étape d) du procédé.

6. Procédé selon l'une ou l'ensemble des revendications 1 à 5, caractérisé en ce qu'en vue de l'élimination des particules tissulaires insuffisamment fixées, le support est traité après l'étape d) du procédé avec un courant de solution de rinçage orienté parallèlement à la surface traitée.

7. Dispositif pour la mise en application du procédé selon la revendication 1, caractérisé en ce qu'il présente un récipient (9) doté de moyens d'agitation (10) pour la digestion enzymatique de particules de tissu, avec une entrée dans la région de laquelle est disposé un moyen (1, 3, 5, 7) pour le hachage mécanique de tissu en particules de tissu, et avec une sortie qui est disposée dans la partie inférieure du récipient (9) dans le sens de la gravité, en ce qu'il présente des moyens(25) pour la filtration d'une suspension de particules de tissu à travers un support artificiel poreux préformé et pour le rinçage du support dans le même sens, la sortie du récipient (9) et les moyens(25) pour la filtration étant reliés entre eux par une conduite par laquelle une suspension de particules de tissus peut être acheminée et dans lequel il est prévu entre le récipient (9) pour la digestion enzymatique et les moyens (25) pour la filtration une unité de filtrage (15) destinée à retenir des particules qui constitueraient un risque pour un patient du fait de leur taille, le dispositif constituant un système pouvant être fermé, sans moyens de centrifugation pour la séparation de la suspension digérée entre le récipient (9) de digestion enzymatique et les moyens (25) de filtration.

8. Dispositif selon la revendication 7, caractérisé en ce que l'unité de filtrage (15) est disposée dans un autre récipient (17) pourvu d'un agitateur (18).

9. Dispositif selon l'une quelconque des revendications 7 ou 8, caractérisé en ce que les moyens d'agitation (10, 18) sont des agitateurs magnétiques.

10. Dispositif selon l'une ou l'ensemble des revendications 7 à 9, caractérisé en ce que les conduites entre le récipient (9) et les moyens (25) de filtration sont des tuyaux à usage unique et en ce que des pompes péristaltiques (13, 19) sont prévues pour le transport de la suspension.

11. Utilisation du procédé et du dispositif selon l'une ou l'ensemble des revendications 1 à 10 pour l'obtention de prothèses vasculaires.

12. Utilisation du procédé et du dispositif selon l'une ou l'ensemble des revendications 1 à 10 pour l'obtention de valvules mitrales.
